# EUROPEAN PATENT SPECIFICATION

(11) **EP 2 768 345 B1**
(45) Date of publication and mention of the grant of the patent: **15.05.2019**
(21) Application number: 12781007.5
(22) Date of filing: 19.10.2012
(51) Int. Cl.: A47D 9/02

(54) **INFANT CALMING/SLEEP-AID DEVICE**
VORRICHTUNG ZUM BERUHIGEN UND IN-DEN-SCHLAF-WIEGEN VON KLEINKINDERN
DISPOSITIF D'AIDE AU SOMMEIL/À LA TRANQUILLISATION DE BÉBÉ

(30) Priority: 20.10.2011 US 201161549627 P
(43) Date of publication of application: 27.08.2014
(73) Proprietor: Happiest Baby, Inc., Los Angeles, CA 90049 (US)
(72) Inventor: KARP, Harvey, Neil, Los Angeles, CA 90049 (US); BERLIN, Matthew, R., Somerville, MA 02144 (US); GRAY, Jesse, V., Somerville, MA 02144 (US); WASHABAUGH, Bill, Walter, Brooklyn, NY 11231 (US); ROY, Deb, Kumar, Belmont, MA 02478 (US)
(74) Representative: Barker Brettell LLP
(86) International application number: PCT/US2012/061069
(87) International publication number: WO 2013/059625

(56) References cited:
- WO-A1-2010/098702
- FR-A1- 2 669 201
- GB-A- 2 312 374

## Description

### BACKGROUND OF THE INVENTION

Persistent crying and poor infant sleep are perennial and ubiquitous causes of parent frustration. During the first months of life, babies fuss/cry an average of about 2 hours/day and wake about 2 to 3 times a night. One in six infants are brought to a medical professional for evaluation for sleep/cry issues.

Infant crying and parental exhaustion are often demoralizing and lead to marital conflict, anger towards the baby and impaired job performance. In addition, they are primary triggers for a cascade of serious/fatal health sequelae, including postpartum depression (which affects about 15% of all mothers and about 25 to about 50% of their partners), breastfeeding failure, child abuse and neglect, suicide, SIDS/suffocation, maternal obesity, cigarette smoking, excessive doctor visits, overtreatment of infants with medication, automobile accidents, dysfunctional bonding, and perhaps infant obesity.

Traditional parenting practices have utilized swaddling, rhythmic motion and certain sounds to soothe fussing infants and promote sleep (by reducing sleep latency and increasing sleep efficiency). "Sleep latency" is defined as the length of time between going to bed and falling asleep. "Sleep efficiency" is defined as the ratio of time spent asleep (total sleep time) to the amount of time spent in bed. Swaddling, rhythmic motion and certain sounds imitate elements of a baby's in utero sensory milieu and activate a suite of brainstem reflexes, the *calming reflex.* Swaddling is a method of snug wrapping with the arms restrained at the baby's sides. This imitates the tight confinement babies experience in the womb. Swaddling also inhibits startling and flailing, which often interrupts sleep and starts/exacerbates crying.

Rhythmic motion replicates the movement fetuses experience when the mother is walking. The motion stimulates the vestibular apparatus in the semicircular canals of the inner ear. A specific, rumbling low-frequency noise imitates the sound created by the turbulence of the blood flowing through the uterine and umbilical arteries. In utero the sound level babies hear has been measured at between 72 and 92 dB. Each baby has a specific and distinctive unique mix of motion and sound that most efficiently activates his or her *calming reflex.* This preferred mix stays consistent through the first months of life (i.e. babies who respond best to swaddling plus motion continue to respond to those modalities over time and don't abruptly switch their preference to swaddling plus sound).

The *calming reflex* has several constant characteristics. It is triggered by a stereotypical sensory input; produces a stereotypical behavioral output; demonstrates a threshold phenomenon (i.e. stimuli that are too mild may not be sufficient to activate a response); has a threshold that varies between individuals (i.e. is higher or lower for any given child); the threshold varies by state (e.g. fussing and crying raise the level of stimulation required to exceed threshold and bring about reflex activation).

Since the nominal level of a stimulus needed to reach the triggering threshold of the *calming reflex* differs from one child to the next, failure to exceed a particular child's threshold level often results in a total absence of a calming response. For example, slow smooth motion may calm one upset infant, yet be too subdued to calm another. Likewise, moderately loud sound may reach the calming threshold for one child, but not another. Once triggered, the stereotypical output of the *calming reflex* is a reduction of motor output and state. The intensity of sound and motion needed to trigger any particular baby's *calming reflex* is much greater than the levels needed to keep the *calming reflex* activated. "State" describes an infant's level of attention to and interaction with the environment. Infants experience six states: quiet sleep, active sleep, drowsiness, quiet alert, fussing and crying.

However, despite the convenience and availability of swaddling, rhythmic motion and sound, these methods fail to calm and promote sleep in a large portion of the infant population because they are not being applied correctly. To reduce infant crying and promote sleep parents often bring the baby into their own bed. However, this is problematic because sharing a bed with a parent has been proven to raise an infant's risk of Sudden Infant Death Syndrome (SIDS) and accidental suffocation (which has been increasing by 14% per year for approximately twenty years). The hazard of bed sharing is further elevated if the parent is extremely fatigued. Like inebriation, exhaustion reduces adult judgment and responsiveness. Over 50% of new parents report sleeping fewer than 6 hours/night, the level demonstrated in adults to simulate a level of attention impairment comparable to inebriation. For this reason, sleeping with an exhausted parent further increases the SIDS risk associated with bed sharing and further increases the suffocation risk (e.g. from accidental overlaying of the parents body over the infant's head).

Other behaviors that exhausted parents engage in to calm crying and promote sleep also directly raise the risk of SIDS and suffocation (e.g. falling asleep with the baby on a couch, placing the baby on the stomach to sleep). Medical authorities recommend parents avoid bed sharing and place sleeping babies in cribs. However, cribs are problematic. Babies sleeping supine in cribs have a higher risk of plagiocephaly (flattening of the skull), which may require expensive and inconvenient medical treatment, and may result in a permanent deformity. In addition, a crib's flat, quiet, nonmoving surface is devoid of the swaddling, rhythmic motion and sound that reduce crying, reduce sleep latency and increase sleep efficiency.

In an attempt to improve infant sleep in cribs, parents have employed several methods (prone sleeping, swaddling, rocking motion, sound), however each is problematic. For example, the prone position is associated with a 3-4 fold increased risk of SIDS. Swaddled babies can roll to the stomach position (prone), which is associated with an 8-19 fold increased risk of SIDS. Rocking motion delivery systems (e.g. swings, cradles and hammocks) all present problems. When sitting in a swing, a baby's head can roll forward and create an airway obstruction. Cradles and hammocks require parents to be the motion-powering energy source, and thus can be done for only a limited part of the sleep period. Sound delivery devices (e.g. fans, air filters, hair driers, sound machines and white noise CDs) may be cumbersome and expensive and the volume, quality or frequency profile of the sound they produce may be excessive or too different from in utero sound to be effective.

Over the past twenty years, attempts have been made to engineer technological methods to create infant calming/sleep devices to deliver sound and motion more conveniently. One such device, a motorized cradle, was designed to rock sleeping babies in an arc along the head-foot axis. This product allowed the cradle to come to rest at an angle, in a partial swing position, which resulted in multiple infant deaths. Another device, designed to simulate a car traveling at about 55 miles per hour, is comprised of 2 parts: a vibrating motor that fixes to the underside of the crib and a speaker that fixes to the sidewall. Still another device has a motorized crib that moves back and forth (about 10 cm in each direction along the head-foot axis; each swing lasting about 1.8 seconds). A sensor activates the device's motor for a limited period of time when it detects the infant's cry. Still other devices have introduced sound machines or mats that vibrate for short periods of time to be placed under the baby to encourage sleeping.

These and other current infant calming/sleep devices deliver fixed and unchangeable motion and sound. This is a problem because each baby has a different mix of sound and motion that most efficiently calms the child and promotes sleep. For example, some babies respond best to swaddling plus motion, others swaddling plus sound. Another problem with fixed motion and sound infant calming/sleep devices is that each baby has a unique level of motion and sound that induces calming and sleep most efficiently. For example, slow rocking may reduce sleep latency for one infant, yet be too subdued to do so in another infant. And, quiet sound may be sufficient to increase sleep efficiency for one baby, but not another. Also, the intensity of sound and motion that a baby needs to trigger the *calming reflex* is much greater than the levels needed to keep the *calming reflex* activated.

Still another problem with fixed motion and sound infant calming/sleep devices is that the intensity of the stimuli needed to activate the *calming reflex* and induce calm and sleep varies substantially as a child's state changes. For example, most fussy babies require more vigorous, jiggly motion (with rapid acceleration-deceleration) and more vigorous sound inputs (as loud as a vacuum cleaner - 75 to 80 dB). On the other hand, calm, sleepy babies need less vigorous inputs. Further, current infant calming/sleep devices do not continue all night long; do not deliver optimal sound and motion for triggering the *calming reflex*; do not increase and decrease their sensory input in a step-wise fashion to vary the sensory input intensity to give the baby the most effective level of stimulation; lack the ability to reduce the sensory input over time to wean a baby off the stimuli as he or she ages.

GB2312374 discloses an infant seat or cradle which has a motor causing recriprocal motion thereof on wheels which are guided in a track. Partial actuation of the motor causes the wheels to engage only in a straight central portion of the track whilst full actuation causes the wheels also to engage upturned end portions. Change between the two motions may be controlled by computer in response to signals from a close proximity sound scanner and a movement sensor. If motion or crying is detected then after a predetermined delay motion will be started to settle the infant. After a further predetermined time, a chime or music controlled by the computer may also sound.

FR2669201 (Figure 3) discloses an an infant calming device according to the preamble of claim

### SUMMARY OF THE INVENTION

The invention provides an infant calming/sleep-aid device, as defined in claim 1.

There is disclosed an adaptive calming and sleep aid method, including the steps of moving an infant in a reciprocating manner about an axis that intersects the infant and is orthogonal to a major plane of the surface supporting the infant. At least one of a sound generated by a sound generating device and a reciprocating movement is modulated in an updating and adaptive matter by a logic circuit-controlled actuation in response to at least one of the sound of the infant and the motion of the platform.

The present invention at least in the preferred embodiments has many advantages. For example, the device provides for modulation of reciprocating movement of an infant in an updating and adaptive manner. The rapidly accelerating and decelerating reciprocal motion of the device which induces the infant's head to accelerate and decelerate over a short distance in a safe and specifically controlled manner induces the infant's natural *calming reflex.* The device's specifically designed motion and sound, along with its adaptive control system reduce irritability during awake hours and improve infant sleep (specifically reducing irritability during periods of sleep, reducing sleep latency and increasing sleep efficiency) for babies up to about twelve months old.

### BRIEF DESCRIPTION OF THE DRAWINGS

FIG. 1 is a perspective view of one embodiment of the infant calming/sleep-aid device of the invention, with a depiction of an infant asleep inside the device.
FIG. 2 is a perspective view of the infant calming/sleep-aid device of FIG. 1 with swaddle fastening straps and without an enclosure.
FIG. 3 is a perspective view of the infant calming/sleep-aid device of FIG. 2, showing apparatus beneath the main moving platform in broken lines.
FIG. 4 is a plan view of the apparatus supporting the main moving platform of the infant calming/sleep-aid device of FIG. 3, with the rigid base and main moving platform shown in outline.
FIG. 5 is a side view of the infant calming/sleep-aid device shown in FIG. 4, taken along line 5-5.
FIG. 6 is a side view of the infant calming/sleep-aid device shown in FIG. 4.
FIG. 7 is a perspective view of yet another embodiment of the calming/sleep-aid device of the invention, showing components of the device beneath the main moving platform in broken lines.
FIG. 8 is a plan view of the apparatus supporting the main moving platform of the calming/sleep-aid device of FIG. 7, with the rigid base and main moving platform shown in outline.
FIG. 9 is a side view of the embodiment of the device shown in FIG. 7. FIG. 10 is a schematic representation of one embodiment of a software control system of the invention, along with inputs and outputs of the software control system.
FIG. 11 is a schematic representation of one embodiment of a crying detection module of the invention.
FIG. 12 is a schematic representation of one embodiment of a motion analysis module of the invention.
FIG. 13 is a schematic representation of one embodiment of a behavior state machine module of the invention.
FIG. 14 is a schematic representation of one embodiment of an audio generation module of the invention.
FIG. 15 is a schematic representation of a motion generation module of the invention.
FIG. 16 is a schematic representation of a motion generation module of the invention.

### DETAILED DESCRIPTION OF THE INVENTION

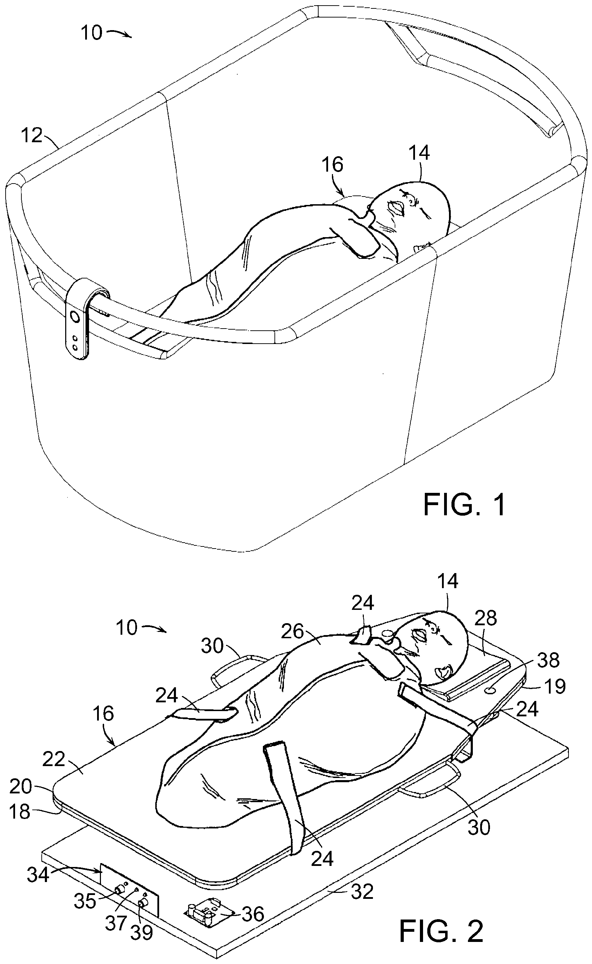

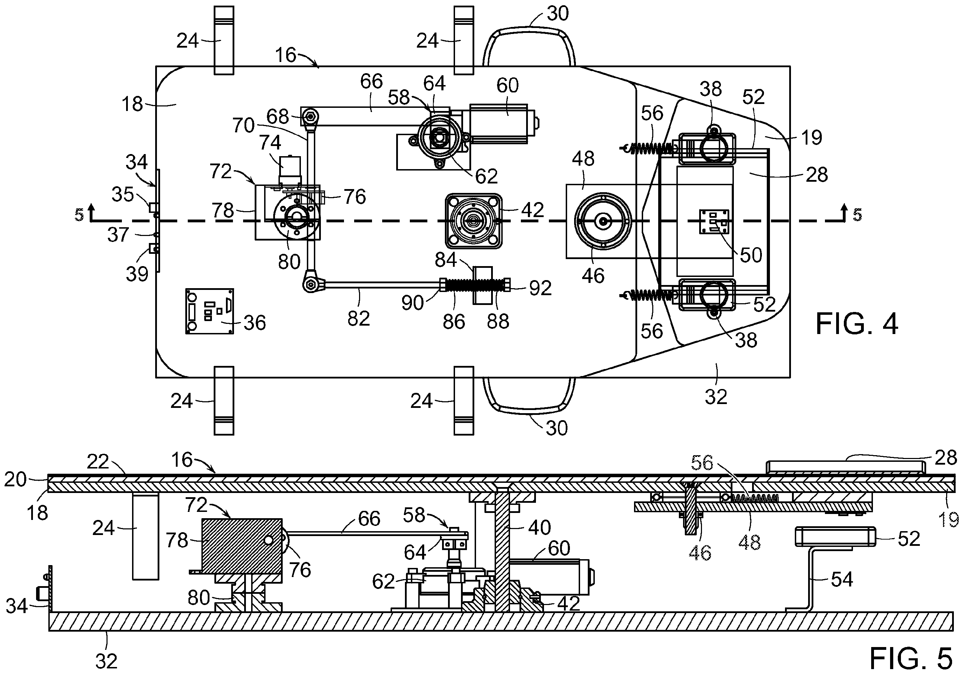

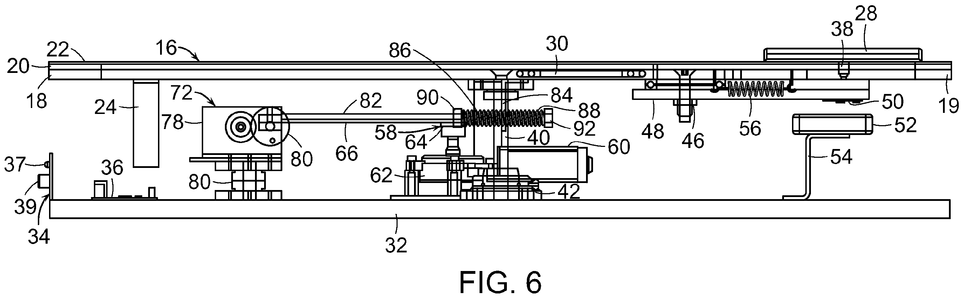

In one embodiment of the invention, shown in FIGs. 1 through 6, infant calming/sleep-aid device **10** includes enclosure **12** about infant **14.** Enclosure **12** surrounds main moving platform **16.** As can be seen in FIG. 2, main moving platform **16** includes base **18,** moving head platform **19,** padding **20** and cloth covering **22.** Swaddle fastening straps **24** extend from main moving platform **16** for securing infant **14** in suitable swaddling clothes **26.** Head pad insert **28** supports the head of infant **14.** Preferably, head pad insert **28** includes a gel in order to reduce the risk of plagiocephaly. Handles **30** extend laterally from main moving platform **16.** Main moving platform **16** is supported and rotatable about a main support shaft (not shown) that is fixed to rigid base **32.** Control panel **34,** which includes speed control knobs **35,** status lights **37** and controls **39** for microphone **38.** Rigid base control electronics **36,** include drive electronics of infant calming/sleep-aid device **10.**

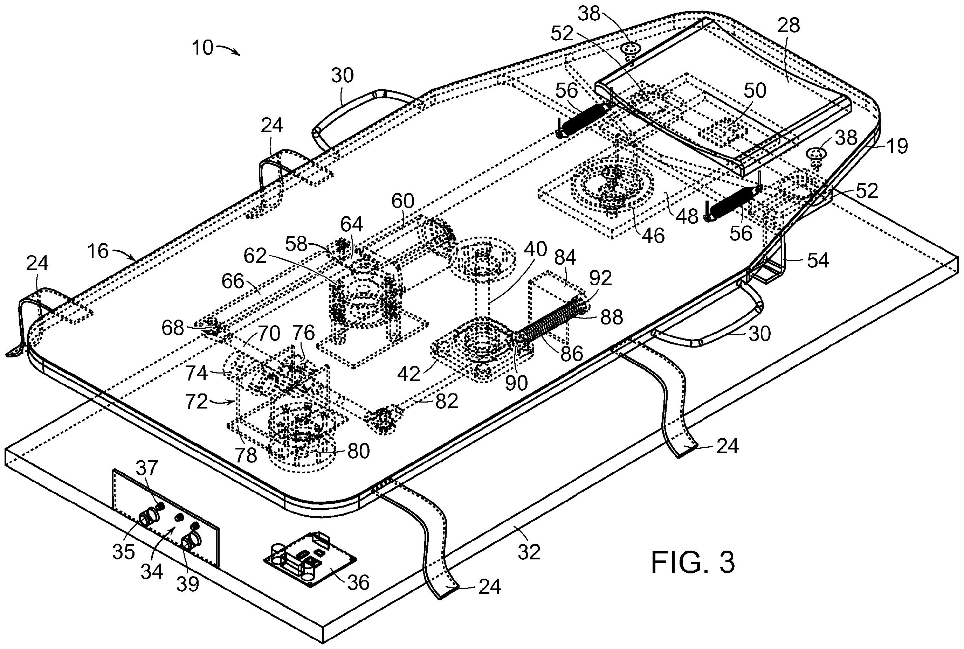

In another representative view of infant calming/sleep-aid device **10** of FIG. 2, shown in FIG. 3, main moving platform **16** is supported by main support shaft **40** at main rotation bearing **42.** Moving head platform **19** supports head pad insert **28** and is rotatable about head rotation bearing **46** through arm **48** extending between head rotation bearing **46** and moving head platform **19.** Motion sensing device **50,** such as an accelerometer, at moving head platform **44** detects motion of moving head platform **19.** Microphones **38** at moving head platform **19** detect sound emitted by the infant (not shown) when supported by infant aid sleep device **10.** Speakers **52,** supported by brackets **54** mounted on rigid base **18,** are located directly beneath moving head platform **19.** Springs **56** linking either side of moving head platform **19** to main moving platform **16** dampen motion of moving head platform **19** relative to main moving platform **16** during reciprocal motion of moving head platform **19** induced by reciprocating motion of main moving platform **16.**

Reciprocating motion of main moving platform **16** about main support shaft **36** is about an axis that is orthogonal to a major plane of main moving platform **16.** Reciprocating motion of main moving platform **16** is driven by actuator assembly **58.**

In some embodiments, the body and the head of the infant can be out of phase. For example, at relatively slow speeds, the motion of the head of the infant can be in the same direction as that of the motion of the upper body of the infant. At relatively high speeds, the reciprocal motion of the head of the infant can be in the opposite direction as that of the upper body of the infant. In another embodiment of the invention (not shown), reciprocal motion of the head of the infant can be in some other direction, such as orthogonally relative to the plane of the main support platform.

Actuator assembly **58** includes drive motor **60** mounted to rigid base **32** and gear assembly **62** linked to drive motor **60** and also mounted to rigid base **32.** Actuation of drive motor **60** causes rotation gear assembly **62** to drive eccentric drive plate **64** about an axis normal to a major plane of rigid base **32.** Eccentric drive plate **64** is linked to swing arm plate **66** of actuator assembly **58** that extends from eccentric drive plate **64** to rod end **68** of screw **70** and is pivotally mounted to rod end **68** of screw **70.** Screw **70** is mounted to amplitude modulation assembly **72.** Amplitude modulation assembly **72** includes amplitude modulation motor **74,** nut **76,** mounted on nut frame **78,** which swivels on rotation bearing **80** mounted to rigid base **32.** The axis of rotation of nut frame **78** on rotation bearing **80** is, like that of eccentric drive plate **64,** normal to a major plane of rigid base **32.** Actuation of amplitude modulation assembly **72** causes movement of screw **70** along its major longitudinal axis to thereby cause rod end **68** to become more proximate or less proximate to amplitude modulation assembly **72.** Arm **82** extends from an end of screw **70** opposite to rod end **68** to elastic actuator catch bracket **84,** which is mounted on base **18** of main moving platform **16.** Arm **82** extends through an opening defined by elastic actuator catch bracket **84** and is linked to main moving platform **16** by springs **86, 88** held in place on either side of elastic actuator catch bracket **84** by nuts **90, 92,** respectively.

Actuation of actuation assembly drive motor **60** causes rotation of eccentric drive plate **64** about an axis normal to a major plane of rigid base **32** which, in turn, causes reciprocal motion of swing arm plate **66** roughly along a major longitudinal axis of swing arm plate **66.** Such reciprocal motion of swing arm plate **66** causes rod end **68** to move in a reciprocal motion from side-to-side of a major longitudinal axis of screw **70** which causes reciprocal rotation of nut frame **80** about an axis normal to major plane rigid base **18** and side-to-side motion of the opposite end of screw **70** opposite that of rod end **68** of screw **70.** Such side-to-side movements of the opposite end of screw **70** causes reciprocal longitudinal movement of arm **82** extending through the opening defined by elastic actuator catch bracket **84.** Resistance to such reciprocal motion of arm **82** causes alternating reciprocal compression and relaxation of springs **86, 88,** which thereby causes reciprocal motion of main moving platform **16** about main support shaft **40** linking main moving platform **16** to rigid base **32.**

The amplitude of reciprocal motion of main moving platform **16** about main support shaft **40** is controlled by the location of screw **70** relative to amplitude modulation assembly **72.** For example, if actuation of amplitude modulation assembly **70** causes rod end **68** to become more proximate to amplitude modulation assembly **70,** the side-to-side motion of the opposite end of screw **70** will become greater, thereby causing the amplification of reciprocal motion of main moving platform **16** about main support shaft **40** to increase. Conversely, actuation of amplitude modulation assembly **72** to cause rod end **68** of screw **70** to become more remote from amplitude modulation assembly **72** will diminish the side-to-side motion of opposite end of screw **70,** thereby reducing the amplitude of reciprocal motion of main moving platform **16** about main support shaft **40.**

Reciprocal motion of main moving platform **16** causes a delayed reciprocal motion of moving head platform **44** about head rotation bearing **46.** The reciprocal motion of moving head platform **44,** although delayed, has greater amplitude about main support shaft **40** because of the rotation of moving head platform **44** about head rotation bearing **46.** However, the amplitude of reciprocal motion of moving head platform **44** about head rotation bearing **46** is dampened by springs **56.** Nevertheless, the reciprocal motion of main moving platform **16** and moving head platform **44** about main support shaft **40** is measured by motion sensing device **50** at moving head platform **44.** Measurements by motion sensing device **50** are relayed back to control panel **34** and rigid base control electronics **36** which, alone, or optionally, in combination with external computer software programming, modulate actuator assembly drive motor **60** and amplitude modulation motor **74.** Motion detection by motion sensing device **50** can also, optionally, modulate computer programming to affect selection and volume of sounds emitted by speakers **52.** Microphones **38,** in addition, or optionally, receive acoustical signals that can be fed back through rigid base control electronics **36** or/and control panel **34** to software, either on-board or remote from infant calming/sleep-aid device **10,** that further modulates actuator assembly drive motor **60,** amplitude modulation motor **74** and/or sounds emitted from speakers **52.** Algorithms associated with modulation of actuator assembly drive motor **60,** amplitude modulation motor **74** and speakers **52** will be more fully discussed below.

In one embodiment, the device allows for a reciprocating motion at 0.5-1.5 cps of ∼2" excursions, but if the baby is fussy the device responds by delivering a smaller excursion (e.g.<1") at a faster rate (∼ 2-4.5 cps). This fast and small motion delivers the specific degree of acceleration-deceleration force to the semicircular canals in the vestibular mechanism of the inner ear that is required to activate the *calming reflex.*

Also, the reciprocating motion typically has a maximum amplitude of less than one inch during the rapid phase of motion (-2-4.5 cps), further ensuring safety of the infant.

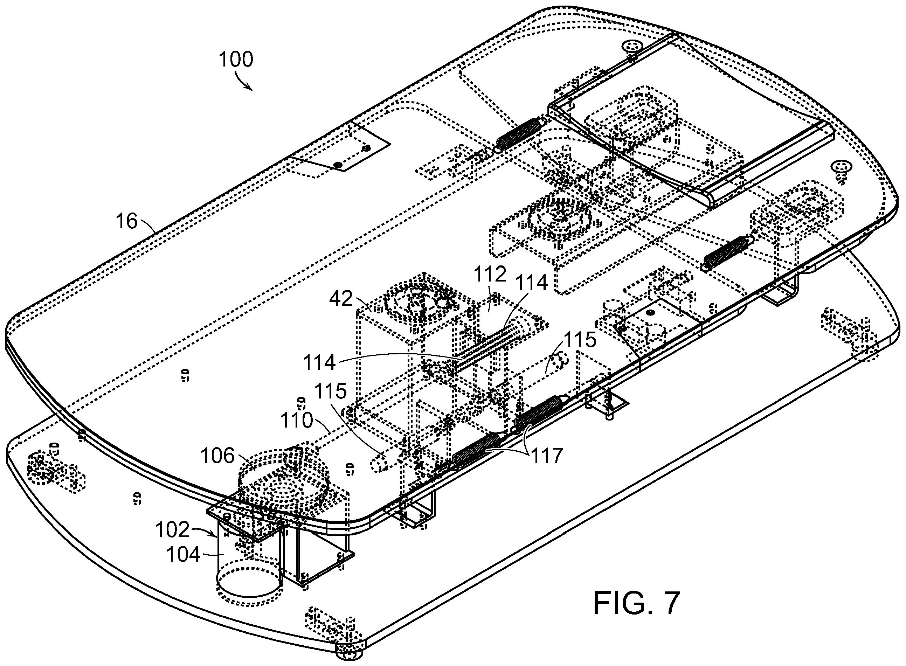

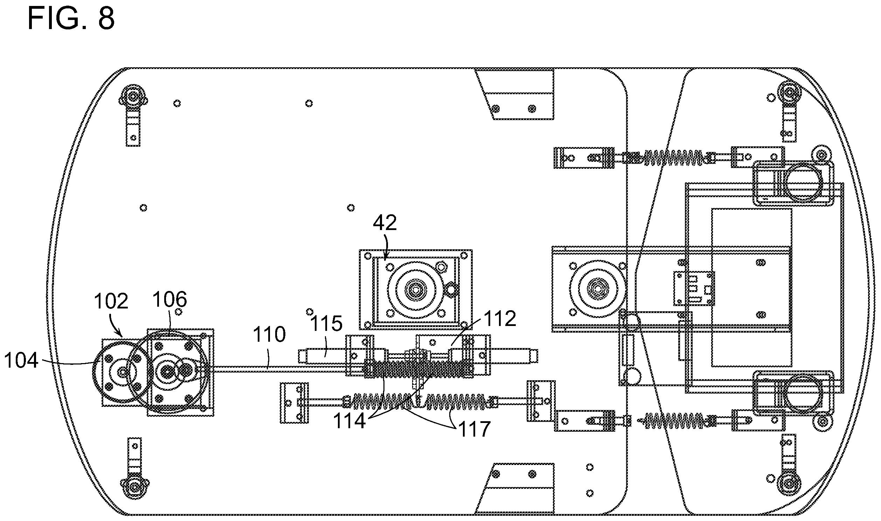

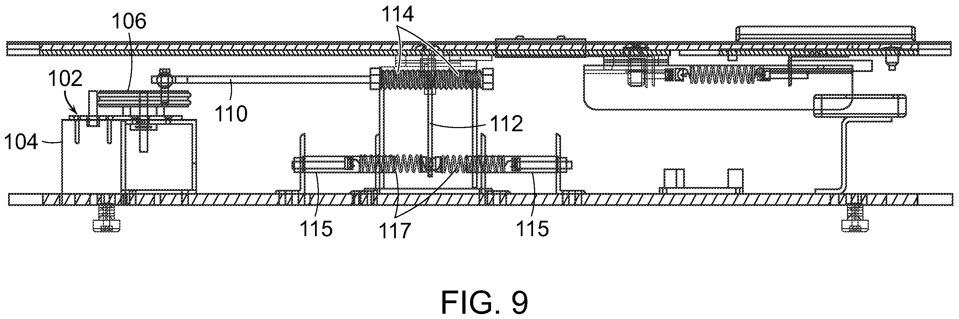

In another embodiment, shown in FIGs. 7 through 9 calming/sleep-aid device **100** includes actuator assembly **102,** which substitutes for actuator assembly **58** of the embodiment shown in FIGs. 2 through 6. Specifically, as shown in FIGs. 7 through 9, drive motor **104** of calming/sleep-aid device **100** is linked to bearing **106,** which is, in turn, leads to the eccentric drive plate **108.** Eccentric drive plate **108** is connected to push/pull rod **110** that extends through an opening defined by elastic actuator catch bracket **112.** Springs **114** about push/pull rod **110** link push/pull rod **110** to main moving platform **16** through elastic actuator catch bracket **112.** Springs **114** are series elastic actuator push-springs; they transfer force from actuator assembly **102** to catch bracket **112.** Balancing dampers **115** beneath push/pull rod **110** dampen the motion of moving platform **16.** Springs **117** are pull-balancing springs; they pull on bracket **112** in parallel with balancing dampers **115** to create the desired smooth sinusoidal motion of moving platform **16** at low frequencies and the rapid accelerating motion at high frequencies.

Actuation of drive motor **104** causes reciprocal longitudinal movement of push/pull rod **110** through the opening defined by elastic actuator catch bracket **112** and translates that reciprocal movement into reciprocal motion of main moving platform **16** about main rotation bearing **42,** as does reciprocal motion of arm **82** through elastic actuator catch bracket **84** of the embodiment shown in FIGs. 2 through 6. Other components of the embodiments shown in FIGs. 7 through 9 operate in the same manner as those of infant calming/sleep-aid device **10** represented in FIGs. 2 through 6.

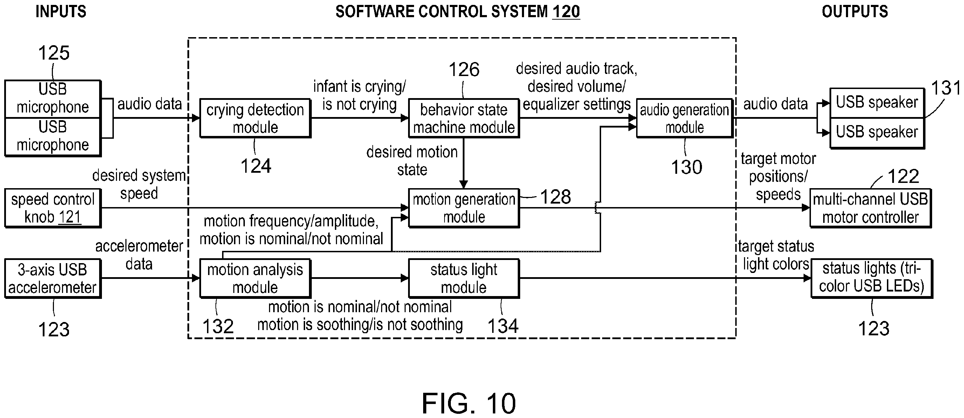

As shown FIG. 10, software control system **120,** processes inputs from microphones and generates outputs to speakers represented in FIGs. 2 through 9, also processes inputs from speed control knob **121,** also shown in FIGs. 2 through 9, and from a three-axis USB accelerometer **123,** represented as motion sensing device in FIGs. 2 through 9. In addition, software control system **120** generates an output signal to multichannel USB motor controller **122,** which controls actuator assembly drive motor **60** (not shown) and amplitude modulation motor **74** (not shown) or, alternatively, as shown in FIGs. 7 through 9, drive motor **104** (not shown). Status lights, such as tricolor USB DEs **121,** are represented as lights **37** in FIGs. 2 through 9. Modules of software control system **120** can be located on-board or remotely from the embodiments of infant calming/sleep-aid devices **10,100** shown in FIGs. 2 through 9. The modules include a crying detection module **124** that receives data from microphones **125,** represented at microphones **38** in FIGs. 2 through 9, and relays to a behavior state machine module **126** whether or not an infant on infant calming/sleep-aid device is crying or not crying. Depending upon the input received by behavior state machine module **126,** output signals will control motion generation module **128** or audio generation module **130.** Actuation of motion generation module **128** will modulate the actuator assemblies of the embodiments shown in FIGs. 2 through 9. Alternatively, or in addition, output signals from behavior state machine module **126** will modulate generation of audio data output from audio generation module **130** to speakers **131,** represented as speakers **52** in FIGs. 2 through 9.

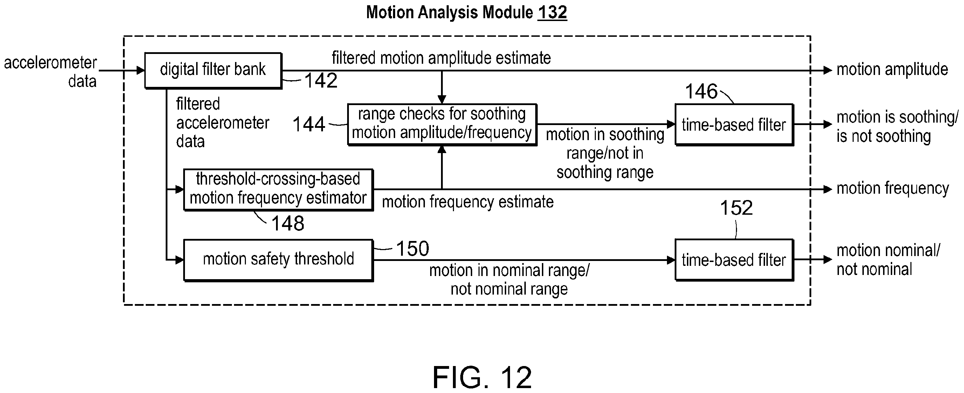

Data received from accelerometer **123** is processed by motion analysis module **132** to thereby modulate the actuator assembly through motion generation module **128** and/or audio generation module **130** to thereby control the actuators assemblies or speakers, respectively. In addition, motion analysis module **132** controls status light module **134** to alert, through the status lights, whether motions of the main moving platform and the head platform are nominal or not nominal, or alternatively, through feedback, soothing or not soothing to the infant. "Nominal", as that term is defined herein, refers to any and all motion for which the filtered acceleration signal does not exceed a specified, or predetermined maximum motion threshold for a specific length of time. The process by which the motion analysis module classifies motion as nominal or not nominal is detailed in FIG. 12 and in the accompanying text below.

In one embodiment the rate of the reciprocating rotation is in a range of between about two and about four and one-half cycles per second and an amplitude of the reciprocating motion at a center of a head of the infant is in a range of between about 0.2 inches and about 1.0 inches. In anther embodiment, the rate of reciprocating motion is in a range of between about 0.5 and about 1.5 cycles per second and an amplitude of the reciprocating rotation at a center of the head of the infant is in a range of between about 0.5 inches and about 2.0 inches.

The software weans the infant off the device by incorporating the infant age as a variable used by the behavior state module control system, wherein modulation is further controlled by at least one of the weight of the infant, the age of the infant, and the duration of the detected sounds made by the infant.

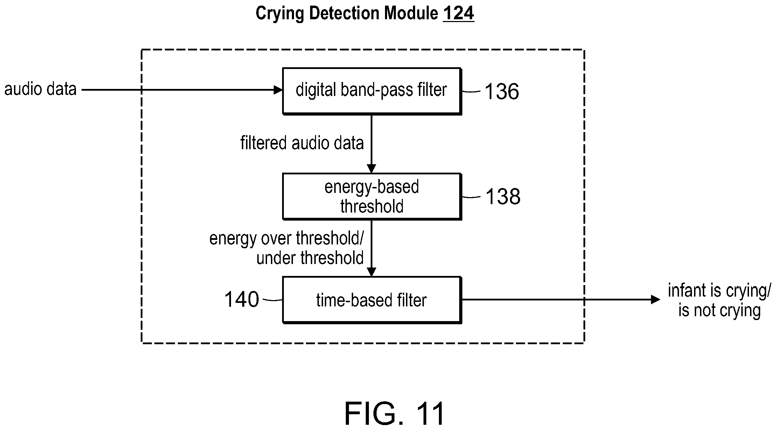

Referring to FIG. 11, crying detection module **124** receives audio data from the microphones of infant calming/sleep-aid devices **10,100,** which is processed through digital band-pass filter **136** to generate filtered audio data. Energy-based threshold **138** receives filtered audio data to determine whether the audio energy is over threshold or under threshold. Time-based filter **140** receives data from energy-based threshold **138** to provide an indication as to whether the infant is crying or not crying. The information, as discussed above with respect to software control system **120** (FIG. 10), is received from crying detection module **124** by behavior state machine module **126** that will then modulate either motion generation module **128** or audio generation module **130.**

Motion analysis module **132,** shown and represented in more detail in FIG. 12, receives a signal from the motion sensing device of infant calming/sleep-aid devices **10,100,** at digital filter bank **142.** Digital filter bank **142** filters the signal to generate a filtered motion amplitude estimate that is used as input to motion generation module **128** (FIG. 10). In addition, the filtered motion amplitude estimate passes through a range check **144** to determine whether the motion is within a soothing or known soothing range. Time-based filter **146** receives data from range check **144** to provide an indication as to whether a motion is soothing or not soothing. Filtered motion sensor, or accelerometer, data from digital filter bank **142** also passes through threshold crossing-based motion frequency estimator **148.** Outputted data from threshold-crossing-based motion frequency estimator **148** passes through range check **144** for indicating whether the motion is or is not soothing, and also provides input to motion generation module **128** (FIG. 10). Filtered accelerometer data from digital filter bank **142** also is processed to determine whether or not the acceleration exceeds a specific maximum motion threshold **150** and, depending on the result, processes that data through time-based filter **152** to provide an indication as to whether the motion is nominal or not nominal. This indication as to whether the motion is nominal or not nominal is used as input to motion generation module **128** (FIG. 10), and is additionally used to control status lights **37** (FIG. 2) via status light module **134** (FIG. 10).

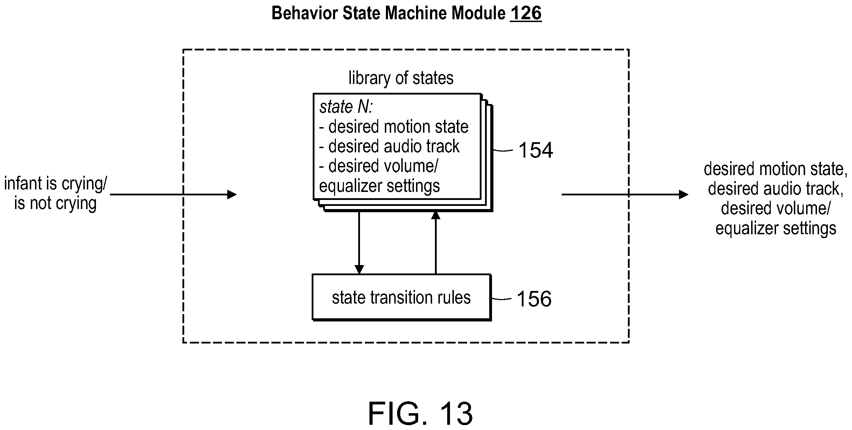

As can be seen in FIG. 13, behavior state machine module **126** receives information from crying detection module **124** (FIG. 11) as to whether the infant is in a state of crying or not crying. This information is used by the state machine's state transition rules **156** to select an active state from a library of states **154,** thereby outputting a desired motion state, a desired audio track and/or desired volume/equalizer settings to audio generation module **130** (FIG. 10).

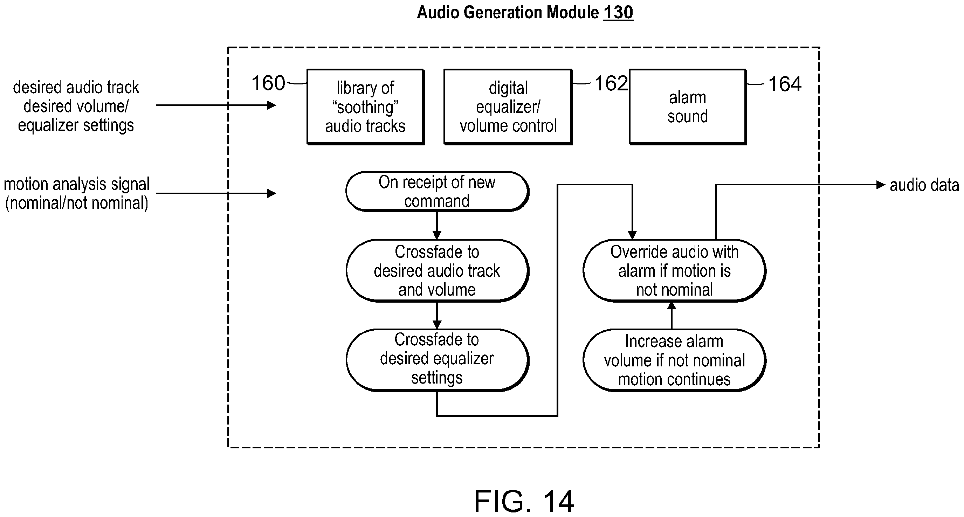

Audio generation module **130,** represented in FIG. 14, receives signals of a desired audio track and desired volume/equalizer settings from behavior state machine module **126** (FIG. 10) and signals of motion analysis, specifically, whether the motion is nominal or not nominal, from motion analysis module **132** (FIG. 10). Audio generation module **130** includes a library of "soothing" audio tracks **160,** a digital equalizer/volume control **162** and alarm sound **164.** Upon receipt of a new command from motion analysis module **132** (FIG. 10), audio generation module **130** will cross-fade to a desired audio track and volume, and crossfade to desired equalizer settings. If the motion is not nominal, then an alarm signal will be output to override the audio signal with an alarm. The audio signal from the audio generation module is output to the speakers of infant calming/sleep-aid device **10,100.**

At baseline, the audio generator will produce an output of a low-pitch, rumbling sound at about 65 dB to about 70dB. Upon receipt of a new command from crying detection module **124** (FIG. 11), audio generation module **130** will cross-fade to a more high pitched audio track and louder volume, at about 75dB to about 80dB.

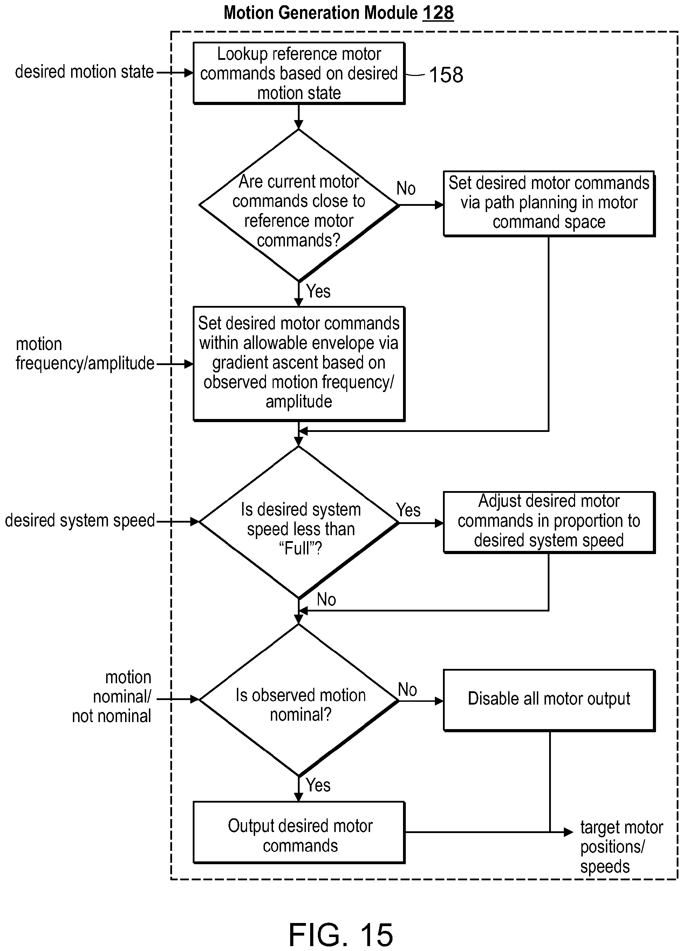

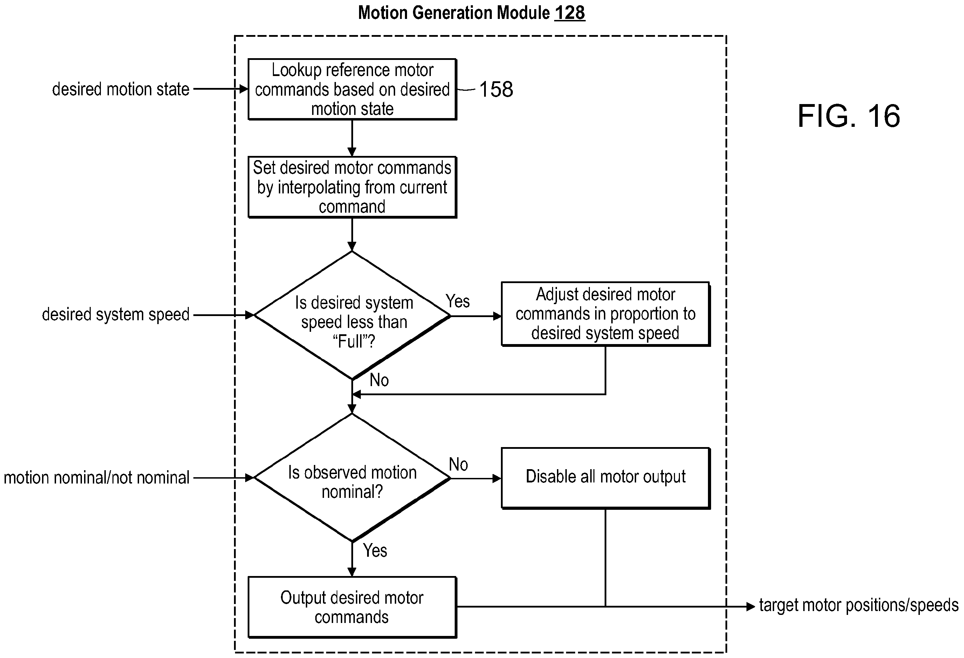

Two variations of motion generation module are represented in FIGs. 15 and 16. In the first embodiment, shown in FIG. 12, motion generation module **128** receives a desired motion state input from behavior state machine module **126** (FIG. 10), a motion frequency/amplitude signal from motion analysis module **132** (FIG. 10), a desired system speed signal from speed control knob **121** (FIG. 10), and a signal as to whether a motion is nominal or is not nominal. The "desired system speed" is the setting of speed control knob 121, whereby the operator can limit the motions allowed by infant calming/sleep-aid device **10,100.** The desired motion state signal goes to lookup within motion generation module **128,** which outputs a reference motor command based on a desired motion state. If the currently-active motor commands are close to the reference motor commands, then the motor commands are actively adjusted within an allowable envelope via a gradient ascent based on observed motion frequency and amplitude. If the current motor commands are not close to the reference motor commands, then the motion generation module will set desired motor commands via path planning in a motor command space. "Path planning" transitions motor settings to desired motor settings by inserting intermediate motor settings as necessitated by nest dynamics to ensure that motion stays in a desirable range during transition. If the desired system speed is less than "full," then a signal is sent to adjust the desired motor commands in proportion to the desired system speed. "Full" is the fully-on position of the knob, and means that infant calming/sleep-aid device **10,100** is not being limited by this knob and is allowed to perform all of the motions it determines to be relevant. If speed control knob 121 is turned down from "full," motions of infant calming/sleep-aid device **10,100** start to become constrained, so speed control knob 121 acts as an operator to override the normal motion behavior of infant calming/sleep-aid device **10,100.** If not, then a comparison is made as to whether the observed motion is nominal. If it is not, then motor output is disabled. If it is nominal, then an output signal of desired motor commands is given to target motor positions and speeds of the actuator of the multichannel USB motor controller.

In an alternative embodiment of motion generation module **128,** shown in FIG. 16, there is no receipt by the module of signals related to motion frequency and amplitude. Therefore, it is only necessary to set desired motor commands by interpolating from a current command based on a look up table of motor commands based on a desired motion state in response to receiving a signal with respect to the desired motion state. All of the components of motion generation are the same as represented in FIG. 15.

### EXPERIMENTAL SECTION

### Materials

Two versions of the infant calming/sleep-aid device as shown in FIGs. 2 through 9 of the invention were created, with microphones to detect infant crying, motion and sound actuators, a swaddling system to keep the baby in optimal position and a gel pad to reduce the pressure on the back of the skull that predisposes to plagiocephaly. The device also contained a logic board to accomplish two tasks; deliver staged interventions of specially engineered sound and to deliver motion created by two linked platforms attached to a motor and rod actuator (as well as a series of springs and dampeners to modulate the activity.) These platforms acted in a reciprocating manner about an axis that intersected the infant and was orthogonal to a major plane of the surface supporting the infant to provide a motion that varies from slow smooth rocking (0.5-1.5 cps) to keep babies calm- and promote sleep...ramping up to a faster, smaller, "jiggly" motion (2-4.5 cps) with a more "spiked" waveform to deliver a sufficiently abrupt acceleration-deceleration action to stimulate the vestibular mechanism of the inner ear, trigger *calming reflex* and soothe the baby when she cried (head rocking back and forth in excursions of less than 1"). The sound in the device was also designed to respond to the baby's upset by starting a specially engineered loud, harsh and high pitched and then stepping down to quieter, lower pitched white noise over several minutes. The device was specifically designed to gradually reduce ("wean") the intensity of the sound and motion over several months.

The device worked in the following way:
The baby was placed in a swaddling sack (with arms in or out) attached to the mattress of the device and securely laid on his/her back. The device produced a baseline level of low pitched, rumbling noise at approximately 65 dB and baseline motion of a smooth, side-to-side rocking (2 inch excursions to either side). When the baby cried for more than ^{∼}10 seconds, the device responded by playing a specially engineered sound that was harsher, higher pitched, more multi-frequency (75-80 dB) to mimic the intensity of the sound that the baby heard inside the mother's uterus prenatally. (This sound can be measured in situ at up to 92 dB.) If the crying continued another ^{∼}10 seconds (despite the sound), the motion accelerated to a faster, more jiggly action of the head (2-4.5 cps, but no more than 1 inch head excursions to either side). The combination of fast movements delivered with sufficient vigor, the harsh loud sound, and the secure swaddling sack all worked together to activate the *calming reflex,* in the majority of irritable babies and inducing either calmness or sleep. The device responded to the baby's cry in a step-wise fashion-gradually increasing sound and then motion-to a maximal level. Once the baby was calmed the motion and sound of the device was gradually reduced in a specific, step-wise fashion back to the baseline activity.

### Subjects

The device was tested on over twenty babies (12 girls, 10 boys) were in the device. The babies ranged from 5 weeks to 6 months of age. Their weights ranged from 8 pounds to 18 pounds.

### Methods and Procedures

The subjects were tested to record their resting and sleeping in the device. The tests usually began when the baby was hungry and tired (immediately before their usual naptime). The parents were introduced to the device and given a brief demonstration of how it worked. We recorded when the baby last fed and napped and then put the baby in the swaddling sack and placed the infant in the device. We observed and videotaped the session. In addition, we collected data from 3 accelerometers and a device-mounted camera to detect the vigor of activity and measure the exact excursions of the baby's head. We started each test with the device set at its lowest level for sound and motion. We observed as the device responded to the baby's cries. We allowed the device to quickly advance through each of its stages as the cries escalated. Once the baby was calmed, the device's motion would slow, in a stepwise fashion, and the loudness and pitch of the sound would decrease, in a stepwise fashion. We repeated this format 2-4 times during our sessions with each of the subjects. The first set of studies was done using a prototype with a dual motion actuator and the second set of studies was done with a prototype with a single motion actuator.

### Results

As shown on the attached table, during twenty-one tests, 19 babies were either significantly calmed or put to sleep by our device (absence of calming was due to hunger). Most calming and sleep occurred within 2 minutes of placing the baby in the device.

We hypothesized that a device could be built that responded to the baby's needs,

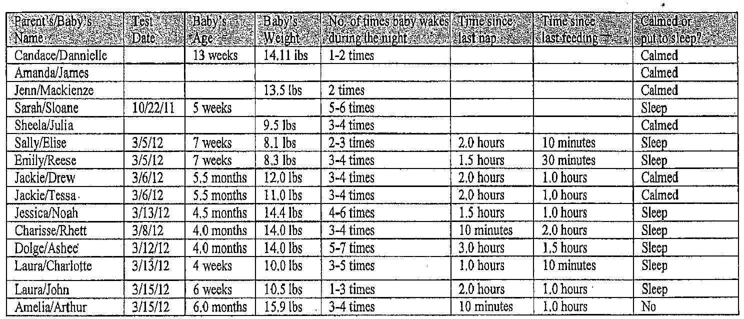

such that an infant's upsets would be soothed by vigorous stimulation to activate the *calming reflex,* followed by a diminution of those stimuli to help keep the *calming reflex* turned on and sustain the baby in a calm state and/or promote sleep (i.e. reducing sleep latency and increasing sleep efficiency.

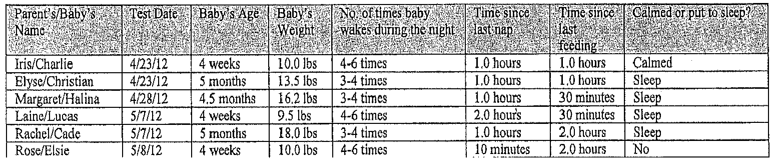

("Sleep latency" is defined as the length of time between going to bed and falling asleep. "Sleep efficiency" is defined as the ratio of time spent asleep - total sleep time - to the amount of time spent in bed.)

### Conclusion

It was possible to promote infant calming and sleep through the use of swaddling plus very specific sound and motion stimuli to activate the *calming reflex.*

### Equivalents

While this invention has been particularly shown and described with references to example embodiments thereof, it will be understood by those skilled in the art that various changes in form and details may be made therein without departing from the scope of the invention encompassed by the appended claims.

## Claims

1. An infant calming/sleep-aid device, comprising:
a) a rigid base (32);
b) a main movement linkage or bearing (42) extending from the rigid base (32);
c) a main moving platform (16) mounted on the main movement linkage or bearing (42), whereby the main moving platform (16) is moveable on the main movement linkage or bearing (42) relative to the rigid base (32); and
d) an actuation assembly (58, 102) that controls movement of the main moving platform (16) about the main movement linkage or bearing (42) relative to the rigid base (32), the actuation assembly (58, 102) including an actuator (60, 104) mounted to the rigid base (32), and the actuation assembly (58, 102) being operable to move the infant in a reciprocating manner about an axis that intersects the infant at a 90 degree angle to a major plane of a surface supporting the infant;
**characterized in that** the rate and amplitude of the reciprocating rotation are controlled by a logic circuit in response to signals obtained from a motion detection device (50, 123) that is connected to the logic circuit and that detects perturbations caused by the infant during reciprocating rotation and/or in response to sounds detected by at least one sound detection device (38, 125) connected to the logic circuit.

2. The infant calming/sleep-aid device of claim 1, further including:
a) a moving head platform (44); and
b) a head movement linkage or bearing (48) mounted to the main moving platform (16) linking the main moving platform (16) to the moving head platform (44).

3. The infant calming/sleep-aid device of claim 1, further including a sound generating device that includes a speaker (131) for generating a desired audio track in response to signals detected by the at least one of a motion sensing device (50, 123) and a sound sensing device (38, 125) monitoring an infant.

4. The infant calming/sleep-aid device of claim 1, wherein the motion detection device includes an accelerometer (123) and the sound detection device includes a microphone (38, 125).

5. The infant calming/sleep-aid device of claim 4, further including an amplitude modulation motor (74), wherein the amplitude modulation motor (74) controls the amplitude of reciprocating rotation of the main moving platform (16) about the main movement linkage or bearing (42).

6. The infant calming/sleep-aid device of claim 5, wherein the actuation assembly includes a drive motor (60, 104), and the device further includes means for controlling the drive motor, and means for controlling the amplitude modulation motor, and wherein the means for controlling the drive motor and the means for controlling the amplitude modulation motor are linked by the logic circuit.

7. The infant calming/sleep-aid device of claim 6, wherein at least one of the accelerometer (123) and the microphone (38, 125) are linked to the drive motor (60, 104) and to the amplitude modulation motor (74) through the logic circuit.

8. The infant calming/sleep-aid device of claim 7, wherein both the accelerometer (123) and the microphone (38, 125) are linked to the drive motor (60, 104) and the amplitude modulation motor (74) through the logic current.

9. The infant calming/sleep-aid device of claim 1, further including a head rest (28), wherein the head rest includes a gel.

10. The infant calming/sleep-aid device of claim 1, further including means (24) for securing the infant placed in a secure swaddling sack to the main moving platform (16).

11. The infant calming/sleep-aid device of claim 1, wherein in one mode, the frequency of the reciprocating rotation is in a range of between about two and about four and one-half cycles per second with an amplitude of the reciprocating rotation at a centre of a head of the infant that is in a range of between about 0.2 inches (0.508 cm) and about 1.0 inches (2.54 cm).

12. The infant calming/sleep-aid device of claim 1, wherein in one mode, the frequency of reciprocating rotation is in a range of between about 0.5 and about 1.5 cycles per second with an amplitude of the reciprocating rotation at a centre of a head of the infant that is in a range of between about 0.5 inches (1.27 cm) and about 1.5 inches (3.81 cm).

13. The infant calming/sleep-aid device of claim 1, wherein the logic circuit is operable to control the frequency of the reciprocating rotation initially to be within a range of between about 0.5 and about 1.5 cycles per second with a corresponding amplitude of the reciprocating rotation at a center of a head of the infant that is in a range of between about 0.5 inches (1.27 cm) and about 1.5 inches (3.81 cm), and if an infant is not soothed, then subsequently control the frequency of the reciprocating rotation to be within a range of between about two and about four and one-half cycles per second with a corresponding amplitude of the reciprocating rotation at a center of a head of the infant that is in a range of between about 0.2 inches (0.508 cm) and about 1.0 inches (2.54 cm).

14. The infant calming/sleep aid device of claim 1, wherein the logic circuit is operable to detect if an infant is being soothed by the reciprocating rotation of the device and, then reduce the reciprocating rotation of the device in a stepwise fashion.

15. The infant calming/sleep-aid device of claim 1, further including a sound generating device that includes a speaker (121) for generating a desired audio track in response to signals detected by the at least one of a motion detection device (50, 123) and a sound detection device (38, 125) monitoring an infant; and wherein if the logic circuit detects that an infant is not being soothed by the device, then the control system is operable to increase a volume and a pitch frequency of the generated audio track.

16. The infant calming/sleep aid device of claim 15, wherein if the logic circuit detects that an infant is being soothed by the device, then the logic circuit controls the sound generating device to reduce the volume of the generated audio track in a stepwise fashion.

17. The infant calming/sleep-aid device of claim 1, wherein the reciprocating rotation is controlled according to the age of the infant, and wherein at a predetermined age of the infant, the reciprocating rotation is reduced.

18. The infant calming/sleep-aid device of claim 1, wherein the reciprocating rotation is controlled by at least one of the weight of the infant, the age of the infant, a duration of detected sound made by the infant, and a duration of detected motion of the infant.

## Patentansprüche

1. Kleinkind-Beruhigungs-/Schlafhilfevorrichtung, umfassend:
a) eine starre Basis (32);
b) ein/e Hauptbewegungsverbindung oder -lager (42), die/das sich von der starren Basis (32) erstreckt;
c) eine Hauptbewegungsfläche (16), die an die/das Hauptbewegungsverbindung oder -lager (42) montiert ist, wodurch die Hauptbewegungsfläche (16) an der/dem Hauptbewegungsverbindung oder -lager (42) bezüglich der starren Basis (32) beweglich ist; und
d) eine Betätigungsanordnung (58, 102), die die Bewegung der Hauptbewegungsfläche (16) um die/das Hauptbewegungsverbindung oder -lager (42) bezüglich der starren Basis (32) steuert, wobei die Betätigungsanordnung (58, 102) einen Stellantrieb (60, 104) enthält, der an der starren Basis (32) montiert ist, und die Betätigungsanordnung (58, 102) betreibbar ist, um das Kleinkind auf eine sich hin- und herbewegende Weise um eine Achse zu bewegen, die das Kleinkind in einem 90-Grad-Winkel zu einer Hauptebene einer Oberfläche, die das Kleinkind trägt, schneidet;
**dadurch gekennzeichnet, dass** die Geschwindigkeit und Amplitude der sich hin- und herbewegenden Drehung durch eine Logikschaltung in Ansprechen auf Signale gesteuert werden, die von einer Bewegungsdetektionsvorrichtung (50, 123) erhalten werden, die mit der Logikschaltung verbunden ist und die Störungen detektiert, die von dem Kleinkind während der sich hin- und herbewegenden Drehung verursacht werden, und/oder in Ansprechen auf Töne, die von mindestens einer Tondetektionsvorrichtung (38, 125) detektiert werden, die mit der Logikschaltung verbunden ist.

2. Kleinkind-Beruhigungs-/Schlafhilfevorrichtung nach Anspruch 1, ferner enthaltend:
a) eine sich bewegende Kopffläche (44); und
b) ein/e Kopfbewegungsverbindung oder -lager (48), die/das an die Hauptbewegungsfläche (16) montiert ist und die Hauptbewegungsfläche (16) mit der sich bewegenden Kopffläche (44) verbindet.

3. Kleinkind-Beruhigungs-/Schlafhilfevorrichtung nach Anspruch 1, ferner enthaltend eine Tonerzeugungsvorrichtung, die einen Lautsprecher (131) enthalten, um einen gewünschten Audiotitel in Ansprechen auf Signale zu erzeugen, die durch eine Bewegungserfassungsvorrichtung (50, 123) und/oder eine Tonerfassungsvorrichtung (38, 125), die ein Kleinkind überwacht, detektiert werden.

4. Kleinkind-Beruhigungs-/Schlafhilfevorrichtung nach Anspruch 1, wobei die Bewegungsdetektionsvorrichtung einen Beschleunigungsmesser (123) enthält und die Tondetektionsvorrichtung ein Mikrofon (38, 125) enthält.

5. Kleinkind-Beruhigungs-/Schlafhilfevorrichtung nach Anspruch 4, ferner enthaltend einen Amplitudenmodulationsmotor (74), wobei der Amplitudenmodulationsmotor (74) die Amplitude der sich hin- und herbewegenden Drehung der Hauptbewegungsfläche (16) um die/das Hauptbewegungsverbindung oder -lager (42) steuert.

6. Kleinkind-Beruhigungs-/Schlafhilfevorrichtung nach Anspruch 5, wobei die Betätigungsanordnung einen Antriebsmotor (60, 104) enthält, und die Vorrichtung ferner eine Einrichtung zum Steuern des Antriebsmotors und eine Einrichtung zum Steuern des Amplitudenmodulationsmotors enthält, und wobei die Einrichtung zum Steuern des Antriebsmotors und die Einrichtung zum Steuern des Amplitudenmodulationsmotors durch die Logikschaltung verbunden sind.

7. Kleinkind-Beruhigungs-/Schlafhilfevorrichtung nach Anspruch 6, wobei der Beschleunigungsmesser (123) und/oder das Mikrofon (38, 125) mit dem Antriebsmotor (60, 104) und mit dem Amplitudenmodulationsmotor (74) durch die Logikschaltung verbunden sind.

8. Kleinkind-Beruhigungs-/Schlafhilfevorrichtung nach Anspruch 7, wobei sowohl der Beschleunigungsmesser (123) als auch das Mikrofon (38, 125) mit dem Antriebsmotor (60, 104) und dem Amplitudenmodulationsmotor (74) durch die Logikschaltung verbunden sind.

9. Kleinkind-Beruhigungs-/Schlafhilfevorrichtung nach Anspruch 1, ferner enthaltend eine Kopfstütze (28), wobei die Kopfstütze ein Gel enthält.

10. Kleinkind-Beruhigungs-/Schlafhilfevorrichtung nach Anspruch 1, ferner enthaltend eine Einrichtung (24) zum Sichern des Kleinkinds, das in einem sicheren Pucksack an der Hauptbewegungsfläche (16) platziert ist.

11. Kleinkind-Beruhigungs-/Schlafhilfevorrichtung nach Anspruch 1, wobei in einem Modus die Frequenz der sich hin- und herbewegenden Drehung in einem Bereich zwischen etwa zwei und etwa viereinhalb Zyklen pro Sekunde liegt mit einer Amplitude der sich hin- und herbewegenden Drehung an einem Mittelpunkt des Kopfes des Kleinkinds, die in einem Bereich zwischen etwa 0,2 Zoll (0,508 cm) und etwa 1,0 Zoll (2,54 cm) liegt.

12. Kleinkind-Beruhigungs-/Schlafhilfevorrichtung nach Anspruch 1, wobei in einem Modus die Frequenz der sich hin- und herbewegenden Drehung in einem Bereich zwischen etwa 0,5 und etwa 1,5 Zyklen pro Sekunde liegt mit einer Amplitude der sich hin- und herbewegenden Drehung an einem Mittelpunkt des Kopfes des Kleinkinds, die in einem Bereich zwischen etwa 0,5 Zoll (1,27 cm) und etwa 1,5 Zoll (3,81 cm) liegt.

13. Kleinkind-Beruhigungs-/Schlafhilfevorrichtung nach Anspruch 1, wobei die Logikschaltung betreibbar ist, um die Frequenz der sich hin- und herbewegenden Drehung zu steuern, um anfänglich innerhalb eines Bereichs zwischen etwa 0,5 und etwa 1,5 Zyklen pro Sekunde zu liegen mit einer entsprechenden Amplitude der sich hin- und herbewegenden Drehung an einem Mittelpunkt des Kopfes des Kleinkinds, die in einem Bereich zwischen etwa 0,5 Zoll (1,27 cm) und etwa 1,5 Zoll (3,81 cm) liegt, und dann anschließend, wenn ein Kleinkind nicht beruhigt wird, die Frequenz der sich hin- und herbewegenden Drehung zu steuern, um innerhalb eines Bereichs zwischen etwa zwei und etwa viereinhalb Zyklen pro Sekunde zu liegen mit einer entsprechenden Amplitude der sich hin- und herbewegenden Drehung an einem Mittelpunkt des Kopfes des Kleinkinds, die in einem Bereich zwischen etwa 0,2 Zoll (0,508 cm) und etwa 1,0 Zoll (2,54 cm) liegt.

14. Kleinkind-Beruhigungs-/Schlafhilfevorrichtung nach Anspruch 1, wobei die Logikschaltung betreibbar ist, um zu detektieren, ob ein Kleinkind durch die sich hin- und herbewegende Drehung der Vorrichtung beruhigt wird, und dann die sich hin- und herbewegende Drehung der Vorrichtung stufenweise zu reduzieren.

15. Kleinkind-Beruhigungs-/Schlafhilfevorrichtung nach Anspruch 1, ferner enthalten eine Tonerzeugungsvorrichtung, die einen Lautsprecher (121) enthält, um einen gewünschten Audiotitel in Ansprechen auf Signale zu erzeugen, die durch eine Bewegungsdetektionsvorrichtung (50, 123) und/oder eine Tondetektionsvorrichtung (38, 125), die ein Kleinkind überwacht, detektiert werden; und wobei, wenn die Logikschaltung detektiert, dass ein Kleinkind durch die Vorrichtung nicht beruhigt wird, das Steuersystem dann betreibbar ist, um eine Lautstärke und eine Tonhöhenfrequenz des erzeugten Audiotitels zu erhöhen.

16. Kleinkind-Beruhigungs-/Schlafhilfevorrichtung nach Anspruch 15, wobei, wenn die Logikschaltung detektiert, dass ein Kleinkind durch die Vorrichtung nicht beruhigt wird, die Logikschaltung dann die Tonerzeugungsvorrichtung steuert, um die Lautstärke des erzeugten Audiotitels stufenweise zu reduzieren.

17. Kleinkind-Beruhigungs-/Schlafhilfevorrichtung nach Anspruch 1, wobei die sich hin- und herbewegende Drehung gemäß dem Alter des Kleinkinds gesteuert wird, und wobei bei einem vorbestimmten Alter des Kleinkinds die sich hin- und herbewegende Drehung reduziert wird.

18. Kleinkind-Beruhigungs-/Schlafhilfevorrichtung nach Anspruch 1, wobei die sich hin- und herbewegende Drehung durch das Gewicht des Kleinkinds, das Alter des Kleinkinds, eine Dauer des detektierten Tons, das von dem Kleinkind stammt, und/oder eine Dauer der detektierten Bewegung des Kleinkinds gesteuert wird.

## Revendications

1. Dispositif d'aide au calme/sommeil d'un nourrisson, comprenant :
a) un socle rigide (32) ;
b) une articulation ou un palier de mouvement principal (42) s'étendant depuis le socle rigide (32) ;
c) une plate-forme mobile principale (16) montée sur l'articulation ou le palier de mouvement principal (42), la plate-forme mobile principale (16) étant mobile sur l'articulation ou le palier de mouvement principal (42) par rapport au socle rigide (32) ; et
d) un ensemble d'actionnement (58, 102) qui contrôle le mouvement de la plate-forme mobile principale (16) autour de l'articulation ou du palier de mouvement principal (42) par rapport au socle rigide (32), l'ensemble d'actionnement (58, 102) comportant un actionneur (60, 104) monté sur le socle rigide (32), et l'ensemble d'actionnement (58, 102) étant utilisable pour faire bouger le nourrisson d'une manière alternée autour d'un axe qui croise le nourrisson à un angle de 90 degrés par rapport à un plan principal d'une surface supportant le nourrisson ;
**caractérisé en ce que** la vitesse et l'amplitude de la rotation alternée sont contrôlées par un circuit logique en réponse à des signaux obtenus à partir d'un dispositif de détection de mouvement (50, 123) qui est relié au circuit logique et qui détecte des perturbations provoquées par le nourrisson pendant une rotation alternée et/ou en réponse à des sons détectés par au moins un dispositif de détection de son (38, 125) relié au circuit logique.

2. Dispositif d'aide au calme/sommeil d'un nourrisson de la revendication 1, comportant en outre :
a) une plate-forme de tête mobile (44) ; et
b) une articulation ou un palier de mouvement de tête (48) monté sur la plate-forme mobile principale (16) reliant la plate-forme mobile principale (16) à la plate-forme de tête mobile (44).

3. Dispositif d'aide au calme/sommeil d'un nourrisson de la revendication 1, comportant en outre un dispositif générant des sons qui comporte un haut-parleur (131) pour générer une piste audio souhaitée en réponse à des signaux détectés par l'au moins un élément parmi un dispositif de détection de mouvement (50, 123) et un dispositif de détection de son (38, 125) surveillant un nourrisson.

4. Dispositif d'aide au calme/sommeil d'un nourrisson de la revendication 1, dans lequel le dispositif de détection de mouvement comporte un accéléromètre (123) et le dispositif de détection de son comporte un microphone (38, 125).

5. Dispositif d'aide au calme/sommeil d'un nourrisson de la revendication 4, comportant en outre un moteur de modulation d'amplitude (74), le moteur de modulation d'amplitude (74) contrôlant l'amplitude de rotation alternée de la plate-forme mobile principale (16) autour de l'articulation ou du palier de mouvement principal (42) .

6. Dispositif d'aide au calme/sommeil d'un nourrisson de la revendication 5, l'ensemble d'actionnement comportant un moteur d'entraînement (60, 104), et le dispositif comportant en outre des moyens pour contrôler le moteur d'entraînement, et des moyens pour contrôler le moteur de modulation d'amplitude, et les moyens pour contrôler le moteur d'entraînement et les moyens pour contrôler le moteur de modulation d'amplitude étant liés par le circuit logique.

7. Dispositif d'aide au calme/sommeil d'un nourrisson de la revendication 6, dans lequel au moins un élément parmi l'accéléromètre (123) et le microphone (38, 125) est lié au moteur d'entraînement (60, 104) et au moteur de modulation d'amplitude (74) par le circuit logique.

8. Dispositif d'aide au calme/sommeil d'un nourrisson de la revendication 7, dans lequel l'accéléromètre (123) et le microphone (38, 125) sont tous deux liés au moteur d'entraînement (60, 104) et au moteur de modulation d'amplitude (74) par le courant logique.

9. Dispositif d'aide au calme/sommeil d'un nourrisson de la revendication 1, comportant en outre une têtière (28), la têtière comportant un gel.

10. Dispositif d'aide au calme/sommeil d'un nourrisson de la revendication 1, comportant en outre des moyens (24) pour attacher le nourrisson placé dans un sac d'emmaillotage sécurisé à la plate-forme mobile principale (16).

11. Dispositif d'aide au calme/sommeil d'un nourrisson de la revendication 1 dans lequel, dans un mode, la fréquence de la rotation alternée se situe dans une gamme comprise entre environ deux et environ quatre cycles et demi par seconde avec une amplitude de la rotation alternée à un centre d'une tête du nourrisson qui se situe dans une gamme comprise entre environ 0,2 pouce (0,508 cm) et environ 1,0 pouce (2,54 cm).

12. Dispositif d'aide au calme/sommeil d'un nourrisson de la revendication 1 dans lequel, dans un mode, la fréquence de rotation alternée se situe dans une gamme comprise entre environ 0,5 et environ 1,5 cycle par seconde avec une amplitude de la rotation alternée à un centre d'une tête du nourrisson qui se situe dans une gamme comprise entre environ 0,5 pouce (1,27 cm) et environ 1,5 pouce (3,81 cm).

13. Dispositif d'aide au calme/sommeil d'un nourrisson de la revendication 1, dans lequel le circuit logique est utilisable pour contrôler la fréquence de la rotation alternée pour qu'elle se situe au départ dans une gamme comprise entre environ 0,5 et environ 1,5 cycle par seconde avec une amplitude correspondante de la rotation alternée à un centre d'une tête du nourrisson qui se situe dans une gamme comprise entre environ 0,5 pouce (1,27 cm) et environ 1,5 pouce (3,81 cm), et si un nourrisson n'est pas apaisé, alors contrôler ensuite la fréquence de la rotation alternée pour qu'elle se situe dans une gamme comprise entre environ deux et environ quatre cycles et demi par seconde avec une amplitude correspondante de la rotation alternée à un centre d'une tête du nourrisson qui se situe dans une gamme comprise entre environ 0,2 pouce (0,508 cm) et environ 1,0 pouce (2,54 cm).

14. Dispositif d'aide au calme/sommeil d'un nourrisson de la revendication 1, dans lequel le circuit logique est utilisable pour détecter si un enfant est apaisé par la rotation alternée du dispositif, et réduire alors la rotation alternée du dispositif par paliers.

15. Dispositif d'aide au calme/sommeil d'un nourrisson de la revendication 1, comportant en outre un dispositif générant des sons qui comporte un haut-parleur (121) pour générer une piste audio souhaitée en réponse à des signaux détectés par l'au moins un élément parmi un dispositif de détection de mouvement (50, 123) et un dispositif de détection de son (38, 125) surveillant un nourrisson ; et dans lequel, si le circuit logique détecte qu'un nourrisson n'est pas apaisé par le dispositif, alors le système de contrôle est utilisable pour augmenter un volume et une fréquence de pas de la piste audio générée.

16. Dispositif d'aide au calme/sommeil d'un nourrisson de la revendication 15 dans lequel, si le circuit logique détecte qu'un nourrisson est apaisé par le dispositif, alors le circuit logique contrôle le dispositif générant des sons pour réduire le volume de la piste audio générée par paliers.

17. Dispositif d'aide au calme/sommeil d'un nourrisson de la revendication 1, dans lequel la rotation alternée est contrôlée en fonction de l'âge du nourrisson, et dans lequel, à un âge prédéterminé du nourrisson, la rotation alternée est réduite.

18. Dispositif d'aide au calme/sommeil d'un nourrisson de la revendication 1, dans lequel la rotation alternée est contrôlée par au moins un paramètre parmi le poids du nourrisson, l'âge du nourrisson, une durée d'un son détecté émis par le nourrisson, et une durée d'un mouvement détecté du nourrisson.
